Europäisches Patentamt

European Patent Office

Office européen des brevets

(11) Publication number: **0 363 876 B1**

## (12) EUROPEAN PATENT SPECIFICATION

(45) Date of publication of patent specification: **23.12.92**   (51) Int. Cl.⁵: **A61K 37/30**, A61K 9/06

(21) Application number: **89118734.6**

(22) Date of filing: **09.10.89**

(54) **Pharmaceutical compositions comprising calcitonin for intra-nasal administration and a spray unit for the administration of the same.**

<table>
<tr><td>

(30) Priority: **11.10.88 IT 2225788**
**08.11.88 IT 2254888**

(43) Date of publication of application:
**18.04.90 Bulletin  90/16**

(45) Publication of the grant of the patent:
**23.12.92 Bulletin  92/52**

(84) Designated Contracting States:
**ES GR**

(56) References cited:
**EP-A- 0 156 772**
**EP-A- 0 193 372**
**GB-A- 2 114 001**

</td><td>

(73) Proprietor: **SCHIAPPARELLI SALUTE S.p.A.**
**Piazza Duca D'Aosta, 12**
**I-20124 Milano(IT)**

(72) Inventor: **Mardente, Salvatore**
**Piazza Duca d'Aosta, 12**
**I-20124 Milano(IT)**
Inventor: **Corneli, Rodolfo**
**Piazza Duca d'Aosta, 12**
**I-20124 Milano(IT)**
Inventor: **Carazzone, Marilena**
**Piazza Duca d'Aosta, 12**
**I-20124 Milano(IT)**

(74) Representative: **Minoja, Fabrizio**
**Studio Consulenza Brevettuale Via Rossini, 8**
**I-20122 Milano(IT)**

</td></tr>
</table>

Rank Xerox (UK) Business Services

## Description

The present invention relates to pharmaceutical compositions comprising calcitonin and to a spray unit for the intranasal administration of said compositions.

More particularly, the invention relates to pharmaceutical compositions whose active ingredient is calcitonin,which compositions are free from preservants and surfactants.

Still more particularly, the invention relates to pharmaceutical compositions for intranasal administration, comprising calcitonin - preferably salmon calcitonin - dissolved in physiological saline adjusted to pH 3.5-4.5 by means of hydrochloric acid.

Calcitonins are known to be of basic importance in the treatment of osteoporosis, Paget's disease, hypercalcemia and similar pathological conditions. Calcitonins are also known to undergo degradation easily, due to the polypeptide nature thereof: as a consequence, they cannot be administered by the oral route. On the other hand, parenteral administration of calcitonins by injection involves remarkable disadvantages, particularly painful reactions in the patient.

Intranasal administration of polypeptide drugs is also widely described in literature; see, e.g.,Felber et al., Experientia, 1969, page 1195; Gennser et al., Lancet, 1974, page 865; Greenberg et al., Antimicrobial Agents and Chemotherapy, 1978, page 596; Bergquist et al., Lancet, 1979, page 215; Pontiroli et al., British Medical Journal, 1982, page 303, besides a number of patents or patent applications concerning intranasal administrations of insulin (EP 94157), vasopressin (Japanese Patent applications 55066-517, 55055-120), polypeptides of various nature (German Patents 2.256.445 and 2.758.483, EP 252, Belgian Patent 860.717, South african Patent 68/4241).

Intranasal administration of calcitonin is in its turn disclosed in various patents. Thus, italian Patent 1.172.324 claims galenic compositions comprising calcitonin characterized in that they contain, besides the active ingredient, benzalkonium chloride or a surfactant, particularly a non-ionic surfactant, or both the above additives. Higher alkanols or sterol polyoxyalkylene ethers are particularly claimed as surfactants.

Both surfactants and benzalkonium chloride are indicated to be necessary ingredients to provide a good calcitonin bioavailability, that is to assure the effective adsorption thereof by nasal mucosa, while benzalkonium chloride would also act as a preservant against pathogen or undesirable micro-organisms.

Similar compositions still containing a quaternary ammonium salt (benzalkonium chloride, cetyltrimethylammonium bromide) are claimed in EP-A-193.372. Japanese Patent Application 61126-034 discloses on its turn nasal formulations of calcitonin containing absorption-increasing agents such as glucose and/or glucosamine;the need for an absorption adjuvant is also stressed by Japanese Patent Application 61118-325, which proposes to this purpose the addition of amino acids, and by EP-A-183.527, which on the contrary suggests the use of benzyl alcohol, ethanol, salicylic acid, capronic acid or polyethylene glycol as absorption enhancers. Moreover, EP-A-111.841 and 115.627 claim intranasal compositions containing calcitonin in admixture with surfactants, among which biliary acids and benzalkonium chloride are disclosed.

Nevertheless, the presence of the latter - and generally of quaternary ammonium salts - is not satisfactory due to the possible undesired effects thereof (Am J Ophthalmol-1988, 105 (6) p 670-3; Contact Dermatitis-1987, 17 (1) p 41-2; Cutis-1987, 39 (5) p 381-3). Therefore, it would be better to provide intranasal compositions of calcitonin free from said salts; however, according to some of the above mentioned documents, said compositions would suffer from poor bioavailability.

All the patent documents reported above completely agree on the need to enhance said bioavailability by means of surfactants of various nature which promote calcitonin absorption through nasal mucosa.

Therefore, it is really surprising the finding by the Applicant that intrasal compositions of calcitonin containing surfactants of various type, or anyhow agents intended to increase absorption by the nasal mucosa, and compositions containing calcitonin alone in a substantially physiological aqueous solution of sodium chloride, practically show no differences in bioavailability nor in preservability.

Therefore, an object of the present invention is provided by pharmaceutical formulations for intranasal administration comprising:

a) a therapeutically effective amount of calcitonin, dissolved in

b) an about 0.9% NaCl solution in purified water adjusted to pH 3.5-4.5.

Preferably, according to the invention, the compositions consist of:

a) a therapeutically effective amount of salmon calcitonin dissolved in

b) an about 0.9% NaCl solution in purified water, adjusted to pH 3.5-4.5 by means of hydrochloric acid.

Moreover, the compositions according to the invention preferably contain 100 to 5.000 I.U., advantageously 250 to 2.000 I.U., of calcitonin per ml of composition.

The advantage from the compositions of the invention, compared to the ones of the prior art, is evident not only since it is possible to avoid quaternary ammonium salts, but also because the addition of the up-to-now suggested calcitonin-absorption enhancer agents can also be avoided.

Said agents, in fact, involve an increase in costs of starting materials and working.

According to a preferred embodiment, the present invention provides also a spray unit intended for spray administration of the above formulations, comprising a bottle (1) for enclosing the composition and a dosing pump fitted to the mouth of the bottle, this spray unit being further characterized in that a head space in said bottle is filled with nitrogen under pressure.

Spray bottles of the above type are known but they suffer from a number of disadvantages in that, for example, problems arise in ensuring tightness to a liquid enclosed therein and especially to any added fat.

The spray bottle according to this invention permits avoiding the problems referred to above and this, especially, owing to a particular construction of its pump which has no suction/delivery valve means (usually of the ball type) associated thereto, and which is designed so as to prevent, in both the inoperative and operative conditions, the content of the bottle from being ejected through the pump.

The pump is, moreover, integrally equipped with a collar packing tightly fitting to the mouth of the bottle thereby to provide a hermetical seal preventing any exchange of gas with the outside, in both the inward and outward direction.

A head space in the bottle is charged with nitrogen which provides an overpressure of 0.2-0.3 bar. This over-pressure is to compensate for liquid withdrawal during spray administration thereby ensuring that the whole of the bottle content is throughly utilized without back-flow of air occurring from the outside. This is of particular benefit if it is thought that the contact of liquid pharmaceutical composition in the bottle with air may result in degradation of the active principle of the composition as well as in bacterial contamination of this latter and, thus, in damage to a person making use thereof.

The bottle is provided with a sump bottom and has a capacity of about 7 ml, and the precompression dosing pump delivers about 100 microliters at each operation which corresponds to a therapeutically effective dose of active principle.

The pump comprises a hollow casing defining a plurality of chambers of varying cross-sections, which is located in the bottle neck with the interposition of said collar packing, and which is secured to the bottle by the aid of a capsule having a bottom member that is rolled around an annular external rim of the bottle neck.

The hollow pump casing has a hollow piston received therein which is spring urged towards a valve seat formed in a piston rod that extends upwardly out of the top of bottle.

Depressing the piston rod causes discharge of liquid enclosed in a toric chamber which is defined between the external surface of the piston and the internal surface of the largest section of pump casing.

Further features of the spray unit of the invention will be better understood when reading the following detailed description of one embodiment thereof shown in the accompanying drawings, wherein

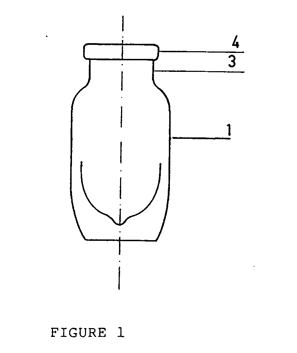

Figure 1 is a side elevational view of a bottle embodying the invention; and

Figure 2 is a cross sectional view shoving on a larger scale a pump as fitted to the neck of the bottle in figure 1.

Referring to the above figures, generally designated with 1 is a glass bottle having a sump bottom 2 and a capacity of preferably about 7 ml.

The bottle 1 is, in a known manner, provided with a neck 3 and the neck 3 has an external raised anular rim, 4 at its top.

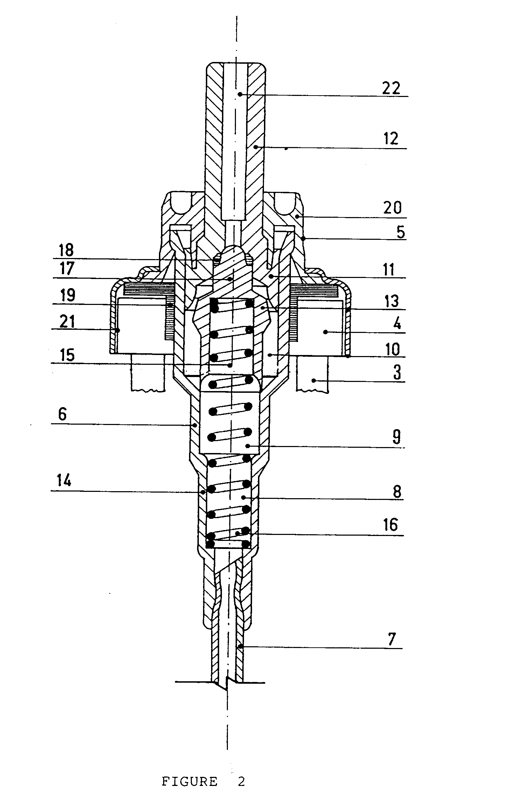

Mounted and sleeved to the bottle 1 is a precompression dosing pump 5 (figure 2) which is designed to deliver about 100 microliters per operation.

The pump 5 comprises a hollow casing 6 fitted in the bottle 1 and carrying at a lower part thereof a plunger member 7.

The hollow casing 6 defines, as seen from bottom to top, three chambers 8, 9 and 10 of increasing sections, and the uppermost of these chambers is arranged to sealingly receive the base 11 of a hollow pistons rod 12 that extends upwardly to project out the top of bottle 1.

Arranged in the pump casing 6 is a piston 13 which is sealingly slidable in the inside of chamber 9 and which is normally urged upwardly by a spring 14 partially located at one end in a recess 15 in piston 13 and resting, at its other end, against an annular shoulder 16 provided in the bottom of the lowermost chamber 8 of casing 6.

The head 17 of piston 13 functions as a valve means and is normally urged by spring 14 towards a valve seat 18 formed in base 11 of piston rod 12.

A collar packing 19 of silicone rubber is interposed between the pump casing 6 and the mouth of bottle 1 and serves as a hermetical seal which besides preventing liquid from flowing out of the bottle, also keeps any exchange of gas from occurring with the outside in neither directions.

The bottle 1 has a capsule 20 of plastic material fitted over its mouth, this capsule being centrally provided with a hole for passage of piston rod 12 and being secured to the bottle by having an aluminium bottom 21 rolled around the annular raised rim 4 of the bottle.

A head space within the bottle 1 is charged with nitrogen to an overpressure of 0.2-0.3 bar.

This overpressure is for the purpose of compressing withdrawal of liquid during utilization so as to ensure thorough use of the whole bottle content, without air backflow from the outside, and, thus, also to ensure that a nitrogen atmosphere is maintained during operation without time limitations.

The nitrogen atmosphere in the bottle is obtained by flushing with nitrogen the empty bottle, distributing the solution, discharging gas from the bottle by vacuum and replacing it by nitrogen, mounting and sleeving the pump, producing overpressure through the pump. All of these operations are accomplished under nitrogen atmosphere.

In this manner, air is nearly thoroughly removed form a head space in the bottle, which head space practically only consists of nitrogen that is maintained over the whole period of validity of product.

In order to use the spray bottle according to the invention, the hollow piston rod 12 is depressed thereby causing the piston 13 to move down against the force of spring 14.

The downward movement of piston 13 results in the toric chamber 10 reducing its volume so that liquid enclosed therein will tend to flow into the head 17 of piston 13 and the valve seat 18 to be then issued as a spray from a passage-way 22 in hollow piston rod 12.

Releasing the piston rod 12 causes the piston 13 and, thus, the piston rod 12 to be urged by spring 14 upwardly to a position as shown in figure 2 which results in further liquid flowing into the toric chamber 10.

The quantity of liquid that is pumped at each delivery, which quantity nearly corresponds to the volume of toric chamber 10, is about 100 microliters.

From the above, it should be apparent that the particular construction of the described pump, which has no suction/delivery valve (usually of the ball type), prevents the bottle content from being ejected through the pump in both the inoperative and operative conditions.

The compositions of the invention were compared with compositions prepared according to the prior art; particularly the pharmacokinetic following intranasal administration of the same dosages (100 I.U.) of salmon calcitonin (salcatonin) in the formulations A and B, in 12 subjects each time:

| A) | Salcatonin | I.U. 100 |
|---|---|---|
| | Sodium chloride | mg 0.9 |
| | 1N hydrochloric acid , q. s. to | pH 4.0 |
| | Purified water, q. s. to | ml 0.1 |

| B) | Salcatonin | U.I. 100 |
|---|---|---|
| | benzalkonium chloride | mg 0.0091 |
| | 1N hydrochloric | mg 0.77 |
| | Purified water, q. s.. to | pH 4.0 |
| | Treatment | ml 0.1 |

Salcatonin plasma concentrations (12 subject mean ± standard error) at various times from administration, are reported in the following Table:

| Minutes | A (pc/ml) | B (pc/ml) |
|---|---|---|
| 0 | 0 | 0 |
| 5 | 25 | 25 |
| 10 | 52 | 55 |
| 20 | 68 | 72 |
| 30 | 38 | 45 |
| 40 | 22 | 20 |
| 50 | 15 | 12 |
| 60 | 10 | 5 |
| 120 | 0 | 0 |

The AUC (area under curve) is 1663 in case of A and 2108 in case of B. $T/2\beta$ is 12.2 minutes and 11.7 minutes, respectively.

The difference between the two curve types is not significant.

Completely analogous results are obtained by comparing the compositions of the invention with compositions containing, instead of benzalkonium chloride, a cholesterol polyoxyethylene ether or respectively ethanol, salicylic acid and sodium taurocholate.

The following examples illustrate the invention in more detail.

**EXAMPLE 1**

Salcatonin (500.000 I.U.) is dissolved in 850 ml of depurated water containing 9 g of analytically pure sodium chloride;pH is adjusted to 4.0 by means of analytically pure 1N HCl and the solution is diluted to 1.000 ml by adding purified water. The solution is filtered on a 0.2 μm filter and distributed in 5 ml containers as disclosed in the annexed figures, provided with a doser delivering 0.1 ml doses each time, equal to 50 I.U. of salcatonin.

**EXAMPLE 2**

The procedure of example 1 is followed, but using a double amount of salcatonin (1.000.000 I.U.). The solution is distributed in suitable 2.5 ml containers as disclosed in the annexed figures delivering 0.1 ml doses each time, equal to 100 U.I. of salcatonin.

**Claims**

1. A process for the preparation of liquid pharmaceutical compositions for intranasal administration, comprising the dissolution of a therapeutically effective amount of calcitonin in a sodium chloride solution in depurated water adjusted to pH 3.5-4.5.

2. A process according to claim 1, in which calcitonin is present in concentrations of 100 to 5.000 I.U. per ml of composition.

3. A process according to claim 2, in wich calcitonin is present in concentrations of 250 to 2.000 I.U. per ml of composition.

4. A process as claimed in claim 1 to 3, in which pH is adjusted to 3.5-4.5 by addition of hydrochloric acid.

5. A process as claimed in claim 1 to 4, wherein salmon calcitonin (salcatonin) is used as the calcitonin.

6. A process as claimed in claim 5 for the preparation of a liquid pharmaceutical composition for intranasal administration, having the following composition:

| | |
|---|---|
| salcatonin | U.I. 500 |
| sodium chloride | mg 9 |
| hydrochloric acid q. s. to | pH 4.0 |
| purified water q. s. to | ml 1.0 |

7. A process as claimed in claim 5 for the preparation of a liquid pharmaceutical composition for intranasal administration, having following composition.

| | |
|---|---|
| salcatonin | U.I.1000 |
| sodium chloride | mg 9 |
| hydrochloric acid q. s. to | pH 4.0 |
| purified water q.s. to | ml 1.0 |

**8.** A spray unit for spray administration of calcitonin by intranasal route, comprising a bottle (1) containing a pharmaceutic liquid composition consisting of a therapeutically effective amount of calcitonin dissolved in a sodium chloride solution in purified water, adjusted to pH 3.5-4.5, and a pump (5) mounted and sleeved to said bottle, the pump (5) comprising a hollow casing (6) formed of a plurality of sections, a piston (13) arranged to operate against the opposition of a spring (14), and a hollow piston rod (12) projecting out the top of bottle (1), characterized in that between the piston (13) and the hollow casing (6) there is defined a toric chamber (10) whose reduction in volume, as a result of the piston rod (12) being acted upon, causes the liquid in said chamber (10) to pass between the piston head (17) and a valve seat (18) formed in a base (11) of piston rod (12), the liquid then being permitted to flow out through a passageway (22) in hollow piston rod (12), and in that a head space in bottle (1) is filled with nitrogen under pressure.

**9.** The spray unit according to claim 8, wherein said head space in bottle (1) is filled with nitrogen to an overpressure of 0.2-0.3 bar.

**10.** The spray unit according to claim 8 or 9, wherein a collar packing (19) is arranged between the hollow casing (6) of pump (5) and the mouth of bottle (1) and functions as a hermetic seal for preventing gas from flowing into, or out the bottle.

**11.** The spray unit according to any one of claims 8 to 10, wherein the bottle (1) has a capacity of about 7 ml and the dosing pump (5) delivers about 100 microliters at each operation, which corresponds to nearly the volume of the toric chamber (10).

**Patentansprüche**

**1.** Verfahren zur Herstellung eines flüssigen pharmazeutischen Präparats für die intranasale Verabreichung, dadurch **gekennzeichnet,** daß es eine therapeutisch wirksame Menge von Calcitonin in Natriumchloridlösung in gereinigtem Wasser, deren pH auf 3,5 bis 4,5 eingestellt ist, gelöst enthält.

**2.** Verfahren nach Anspruch 1, dadurch **gekennzeichnet,** daß das Calcitonin in Konzentrationen von 100 bis 5000 I.E. pro ml Präparat vorhanden ist.

**3.** Verfahren nach Anspruch 2, dadurch **gekennzeichnet,** daß das Calcitonin in Konzentrationen von 250 bis 2000 I.E. pro ml Präparat vorhanden ist.

**4.** Verfahren nach einem der Ansprüche 1 bis 3, dadurch **gekennzeichnet,** daß der pH durch Zugabe von Chlorwasserstoffsäure auf 3,5 bis 4,5 eingestellt wird.

**5.** Verfahren nach einem der Ansprüche 1 bis 4, dadurch **gekennzeichnet,** daß als Calcitonin Lachscalcitonin (Salcatonin) verwendet wird.

**6.** Verfahren nach Anspruch 5 für die Herstellung eines flüssigen pharmazeutischen Präparats für die intranasale Verabreichung, dadurch **gekennzeichnet,** daß es die folgende Zusammensetzung besitzt:

| | |
|---|---|
| Salcatonin | 500 I.E. |
| Natriumchlorid | 9 mg |
| Chlorwasserstoffsäure q.s. auf | pH 4,0 |
| gereinigtes Wasser q.s. auf | 1,0 ml. |

**7.** Verfahren nach Anspruch 5 für die Herstellung eines flüssigen pharmazeutischen Präparats für die intranasale Verabreichung, dadurch **gekennzeichnet,** daß es die folgende Zusammensetzung besitzt:

6

| Salcatonin | 1000 I.E. |
| Natriumchlorid | 9 mg |
| Chlorwasserstoffsäure q.s. auf | pH 4,0 |
| gereinigtes Wasser q.s. auf | 1,0 ml. |

8. Sprüheinheit zur Sprühverabreichung von Calcitonin durch den intranasalen Weg, umfassend eine Flasche (1), die eine pharmazeutische flüssige Zusammensetzung enthält, bestehend aus einer therapeutisch wirksamen Menge von Calcitonin, aufgelöst in einer Natriumchlorid-Lösung in gereinigtem Wasser, eingestellt auf pH 3,5 bis 4,5, und eine Pumpe (5), die an der Flasche angebracht und mit einer Hülse versehen ist, wobei die Pumpe (5) ein hohles Gehäuse (6), das aus einer Mehrzahl von Abschnitten ausgebildet ist, einen Kolben (13), der so eingerichtet ist, daß er gegen den Widerstand einer Feder (14) wirkt, und eine hohle Kolbenstange (12), die aus der Oberseite der Flasche (1) vorsteht, umfaßt, dadurch **gekennzeichnet,** daß zwischen dem Kolben (13) und dem hohlen Gehäuse (6) eine wulstförmige Kammer (10) begrenzt ist, deren Volumenverminderung als ein Ergebnis der Daraufeinwirkung der Kolbenstange (12) bewirkt, daß die Flüssigkeit in der Kammer (10) zwischen dem Kolbenkopf (17) und einem in einer Basis (11) der Kolbenstange (12) ausgebildeten Ventilsitz (18) hindurchgeht, wobei es der Flüssigkeit dann ermöglicht wird, durch einen Durchgang (22) in der hohlen Kolbenstange (12) herauszuströmen, und daß ein Kopfraum in der Flasche (1) mit Stickstoff unter Druck gefüllt ist.

9. Sprüheinheit nach Anspruch 8, worin der Kopfraum in der Flasche (1) mit Stickstoff auf einen Überdruck von 0,2 bis 0,3 bar gefüllt ist.

10. Sprüheinheit nach Anspruch 8 oder 9, worin eine Halspackung (19) zwischen dem hohlen Gehäuse (6) der Pumpe (5) und dem Mund der Flasche (1) angeordnet ist und als eine hermetische Abdichtung zum Verhindern, daß Gas in die oder aus der Flasche strömt, funktioniert.

11. Sprüheinheit nach irgendeinem der Ansprüche 8 bis 10, worin die Flasche (1) eine Kapazität von etwa 7 ml hat und die Dosierpumpe (5) etwa 100 $\mu$l bei jedem Arbeitsvorgang abgibt, was annähernd dem Volumen der wulstförmigen Kammer (10) entspricht.

**Revendications**

1. Procédé de préparation de compositions pharmaceutiques liquides pour administration par voie nasale, comprenant la dissolution d'une quantité thérapeutiquement efficace de calcitonine dans une solution de chlorure de sodium dans de l'eau purifiée, ajustée à pH 3,5-4,5.

2. Procédé selon la revendication 1, dans lequel la calcitonine est présente à des concentrations de 100 à 5000 U.I. par ml de composition.

3. Procédé selon la revendication 2, dans lequel la calcitonine est présente à des concentrations de 250 à 2000 U.I. par ml de composition.

4. Procédé selon l'une des revendications 1 à 3, dans lequel le pH est ajusté à 3,5-4,5 par addition d'acide chlorhydrique.

5. Procédé selon l'une des revendications 1 à 4, dans lequel de la calcitonine de saumon (salcatonine) est utilisée comme calcitonine.

6. Procédé selon la revendication 5 pour la préparation d'une composition pharmaceutique liquide pour administration par voie nasale, ayant la composition suivante :

| Salcatonine | U.I. 500 |
|---|---|
| Chlorure de sodium | mg 9 |
| Acide chlorhydrique   q.s. pour | pH 4,0 |
| Eau purifiée   q.s. pour | ml 1,0 |

7. Procédé selon la revendication 5, pour la préparation d'une composition pharmaceutique liquide pour administration par voie nasale, ayant la composition suivante :

| Salcatonine | U.I. 1000 |
|---|---|
| Chlorure de sodium | mg 9 |
| Acide chlorhydrique   q.s. pour | pH 4,0 |
| Eau purifiée   q.s. pour | ml 1,0 |

8. Ensemble de pulvérisation pour une administration par pulvérisation de calcitonine par voie nasale, comprenant une bouteille (1) contenant une composition liquide pharmaceutique consistant en une quantité thérapeutiquement efficace de calcitonine dissoute dans une solution de chlorure de sodium dans l'eau purifiée, ajustée à pH 3,5-4,5, et une pompe (5) montée et emmanchée sur ladite bouteille, la pompe (5) comprenant un boitier creux (6) formé de plusieurs sections, un piston (13) disposé pour agir à l'encontre de l'action d'un ressort (14), et une tige creuse de piston (12) faisant saillie hors de la partie supérieure de la bouteille (1), caractérisé en ce qu'entre le piston (13) et le boîtier creux (6), il est défini une chambre torique (10) dont la réduction en volume, par suite de l'actionnement de la tige de piston (12), amène le liquide présent dans ladite chambre (10) à passer entre la tête de piston (17) et un siège de soupape (18) formé dans une base (11) de la tige de piston (12), le liquide étant ensuite autorisé à s'écouler à travers un passage (22) dans la tige creuse de piston (12), et en ce qu'un espace de tête dans la bouteille (1) est rempli par de l'azote sous pression.

9. Ensemble de pulvérisation selon la revendication 8, dans lequel ledit espace de tête dans la bouteille (1) est rempli par de l'azote à une surpression de 0,2-0,3 bar.

10. Ensemble de pulvérisation selon la revendication 8 ou 9, dans lequel un collet d'étanchéité (19) est disposé entre le boîtier creux (6) de la pompe (5) et l'embouchure de la bouteille (1) et joue le rôle de joint hermétique pour empêcher du gaz de s'écouler dans la bouteille ou hors de celle-ci.

11. Ensemble de pulvérisation selon l'une quelconque des revendications 8 à 10, dans lequel la bouteille (1) a une capacité d'environ 7 ml, et la pompe de dosage (5) délivre environ 100 microlitres à chaque opération, ce qui correspond approximativement au volume de la chambre torique (10).

FIGURE 1

FIGURE  2